# EUROPEAN PATENT APPLICATION

(11) **EP 4 751 722 A1**
(43) Date of publication of application: **03.06.2026**
(21) Application number: 24844836.7
(22) Date of filing: 25.07.2024
(51) Int. Cl.: A61K 31/519, A61K 31/381, A61K 31/41, A61K 31/495, A61K 31/397, A61K 47/20, A61K 47/10, A61K 47/32, A61K 47/14, A61K 47/22, A61K 9/06, A61K 9/08, A61K 9/10, A61P 17/14

(54) **COMBINED DRUG, AND PHARMACEUTICAL COMPOSITION AND PREPARATION METHOD THEREFOR AND USE THEREOF**

(30) Priority: 25.07.2023 CN 202310923614
(71) Applicant: Beijing Maxinovel Pharmaceuticals Co., Ltd., Beijing 100176 (CN)
(72) Inventor: WANG, Yuguang, Guangzhou, Guangdong 510670 (CN); BI, Lijun, Guangzhou, Guangdong 510670 (CN)
(74) Representative: Michalski Hüttermann & Partner Patentanwälte mbB
(86) International application number: PCT/CN2024/107531
(87) International publication number: WO 2025/021150

(57) **Abstract**

Disclosed in the present invention are a combined drug, and a pharmaceutical composition and a preparation method therefor and a use thereof. The present invention provides the combined drug. Active ingredients of the combined drug comprise an active ingredient A and an active ingredient B, wherein the active ingredient A is a compound as represented by formula I, a pharmaceutically acceptable salt thereof, a solvate thereof, or a solvate of the pharmaceutically acceptable salt thereof, and the active ingredient B is minoxidil, a pharmaceutically acceptable salt thereof, a solvate thereof, or a solvate of the pharmaceutically acceptable salt thereof. The combined drug and the pharmaceutical composition of the present invention have good permeability, stability and safety, have a good therapeutic effect on alopecia areata, and are suitable for topical administration.

## Description

This application claims priority to Chinese Patent Application No. 2023109236146, filed on July 25, 2023. The aforementioned Chinese patent application is incorporated herein by reference in its entirety.

### TECHNICAL FIELD

The present disclosure belongs to the field of pharmaceutical technology, specifically relating to a combined drug, a pharmaceutical composition, and a preparation method therefor and a use thereof.

### BACKGROUND

Alopecia areata (AA) is a common inflammatory non-scarring hair loss condition. The clinical manifestation of this disease is sudden, well-defined circular patchy alopecia on the scalp. Most mild cases can resolve spontaneously, while approximately half of the patients experience recurrent episodes that may persist for several years or even decades. In a minority of patients with severe conditions, hair loss may affect the entire scalp and even extend to body hair across the whole body. The disease can occur at any age, with a higher prevalence among young and middle-aged adults, and no significant gender difference. Epidemiological studies indicate that the prevalence of AA in China is 0.27%, while foreign research suggests a lifetime prevalence of approximately 2% in the general population. This condition affects appearance and can have a negative impact on patients' mental health and quality of life.

Research has found that in patients with alopecia areata, hair follicle cells overexpress NKG2D protein due to genetic factors or external stimuli, leading to attacks on hair follicles by CD4 and CD8 T cells, NK cells, and others. IL-15 secreted by hair follicles activates T cells by binding to JAK1 and JAK3, resulting in upregulation of IFN-γ secretion. After binding to IFN-γ receptors on hair follicle cells, IFN-γ can activate further IL-15 secretion, forming a continuously stimulating loop, and T cells also proliferate continuously. Many cytokines involved in the pathogenesis of autoimmune and inflammatory diseases transmit intracellular signals through the JAK/STAT signaling pathway. Currently, JAK inhibitors have emerged as one of the leading candidate drugs for the treatment of AA. The compound as shown in formula I, i.e. (2-[1-(ethylsulfonyl)-3-(4-{2-[(1-methyl-1H-pyrazol-4-yl)amino]thieno[3,2-d]pyrimidin-4-yl}-1H-pyrazol-1-yl)azetidin-3-yl]acetonitrile) (which is further described in disclosures CN103936757A and WO2014111037A), is a JAK inhibitor that simultaneously inhibits JAK1, JAK2, and JAK3.

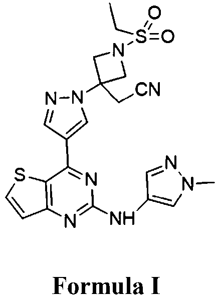

Minoxidil directly stimulates the proliferation and differentiation of hair follicle epithelial cells; promotes angiogenesis and increases local blood flow; opens potassium ion channels; and facilitates the transition of hair follicles from the telogen phase to the anagen phase. It is suitable for AA patients in the stable phase with small areas of hair loss, and requires long-term medication to maintain efficacy. It is often used in combination with other treatments and should be avoided as monotherapy in the progressive phase of AA, and has limited effectiveness for patients with moderate to severe hair loss. The topical concentration of minoxidil is generally 2% and 5%, exhibiting a dose-dependent effect. While a 5% concentration may offer better therapeutic efficacy, it is also associated with a relatively higher incidence of adverse reactions. Adverse reactions are primarily local irritation and hypertrichosis.

Currently, the approved minoxidil formulations for the treatment of hair loss and alopecia areata are all topical preparations. Currently, all approved JAK inhibitors on the market are oral formulations, which are systemic medications that can cause significant side effects.

### SUMMARY

The technical problem to be solved by the present disclosure is the shortage of JAK inhibitor types for treating alopecia areata in the prior art. Thus the present disclosure provides a combined drug, a pharmaceutical composition, a preparation method therefor, and a use thereof. The combined drug and the pharmaceutical composition of the present disclosure have good permeability, stability and safety, have a good therapeutic effect on alopecia areata, and are suitable for topical administration.

The present disclosure provides a combined drug, active ingredients of which comprise an active ingredient A and an active ingredient B;
wherein the active ingredient A is a compound of formula I, a pharmaceutically acceptable salt thereof, a solvate thereof, or a solvate of the pharmaceutically acceptable salt thereof;
the active ingredient B is minoxidil, a pharmaceutically acceptable salt thereof, a solvate thereof, or a solvate of the pharmaceutically acceptable salt thereof;

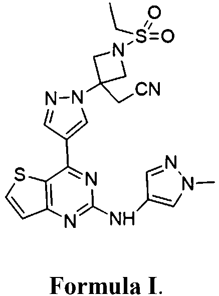

In one embodiment of the present disclosure, the active ingredients consist of the active ingredient A and the active ingredient B.

In one embodiment of the present disclosure, the mass ratio of the active ingredient A to the active ingredient B may be 1:100 to 100:1, preferably 1:10 to 10:1, for example, 1:2, 1:1.7, 1:1.3, 1:1.1, or 1:1.

In one embodiment of the present disclosure, the dosage form of the combined drug may be a paste, a paint, a coating agent, a liniment, an emulsion, an ointment, a cream, a tincture, a patch, a plaster, or a gel; preferably a liniment.

In one embodiment of the present disclosure, the combined drug is a combined drug for preventing, alleviating, or treating alopecia areata.

In one embodiment of the present disclosure, the active ingredient A and the active ingredient B may be administered simultaneously or separately.

The term "administered simultaneously" refers to the administration of the active ingredient A and the active ingredient B contained in one pharmaceutical composition at the same time; or, the administration of "a separate pharmaceutical composition comprising the active ingredient A" and "a separate pharmaceutical composition comprising the active ingredient B" at the same time.

The term "administered separately" refers to the administration of "a separate pharmaceutical composition comprising the active ingredient A" and "a separate pharmaceutical composition comprising the active ingredient B" at different times, for example, one of "the separate pharmaceutical composition comprising the active ingredient A" and "the separate pharmaceutical composition comprising the active ingredient B" is administered first, and the other is subsequently administered. The term "administered separately" may refer to administrations performed at close temporal intervals or at distant temporal intervals.

The present disclosure provides a pharmaceutical composition A, comprising a first pharmaceutical composition and a second pharmaceutical composition;
wherein the first pharmaceutical composition comprises an active ingredient A and a pharmaceutically acceptable carrier, and the active ingredient A is a compound of formula I, a pharmaceutically acceptable salt thereof, a solvate thereof, or a solvate of the pharmaceutically acceptable salt thereof;
the second pharmaceutical composition comprises an active ingredient B and a pharmaceutically acceptable carrier, and the active ingredient B is minoxidil, a pharmaceutically acceptable salt thereof, a solvate thereof, or a solvate of the pharmaceutically acceptable salt thereof;

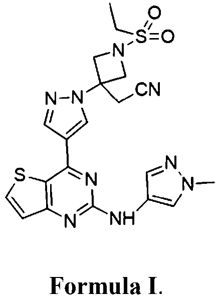

In one embodiment of the present disclosure, the pharmaceutical composition A consists of the first pharmaceutical composition and the second pharmaceutical composition.

In one embodiment of the present disclosure, the first pharmaceutical composition consists of the active ingredient A and the pharmaceutically acceptable carrier.

In one embodiment of the present disclosure, the second pharmaceutical composition consists of the active ingredient B and the pharmaceutically acceptable carrier.

In one embodiment of the present disclosure, the mass ratio of the active ingredient A to the active ingredient B may be 1:100 to 100:1, preferably 1:10 to 10:1, for example, 1:2, 1:1.7, 1:1.3, 1:1.1, or 1:1.

In one embodiment of the present disclosure, the dosage form of the first pharmaceutical composition may be a paste, a paint, a coating agent, a liniment, an emulsion, an ointment, a cream, a tincture, a patch, a plaster, or a gel; preferably a liniment.

In one embodiment of the present disclosure, the dosage form of the second pharmaceutical composition may be a paste, a paint, a coating agent, a liniment, an emulsion, an ointment, a cream, a tincture, a patch, a plaster, or a gel; preferably a liniment.

In one embodiment of the present disclosure, the dosage form of the pharmaceutical composition A may be a paste, a paint, a coating agent, a liniment, an emulsion, an ointment, a cream, a tincture, a patch, a plaster, or a gel; preferably a liniment.

In one embodiment of the present disclosure, the pharmaceutical composition A is a pharmaceutical composition for preventing, alleviating, or treating alopecia areata.

In one embodiment of the present disclosure, the first pharmaceutical composition and the second pharmaceutical composition may be administered simultaneously or separately.

The present disclosure provides a combined drug kit, comprising
a first container, comprising the first pharmaceutical composition as described above; and
a second container, comprising the second pharmaceutical composition as described above.

The present invention provides a pharmaceutical composition B, comprising active ingredients and a pharmaceutically acceptable carrier;
wherein the active ingredients comprise an active ingredient A and an active ingredient B;
the active ingredient A is a compound of formula I, a pharmaceutically acceptable salt thereof, a solvate thereof, or a solvate of the pharmaceutically acceptable salt thereof;
the active ingredient B is minoxidil, a pharmaceutically acceptable salt thereof, a solvate thereof, or a solvate of the pharmaceutically acceptable salt thereof;

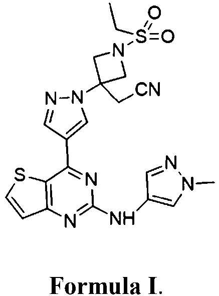

In one embodiment of the present disclosure, the pharmaceutical composition B is a pharmaceutical composition for preventing, alleviating, or treating alopecia areata.

In one embodiment of the present disclosure, the active ingredients consist of the active ingredient A and the active ingredient B.

In one embodiment of the present disclosure, the active ingredients are present in the pharmaceutical composition B in a mass percentage of 0.2% to 20%, preferably 1% to 10%, for example, 3%, 3.2%, 3.5%, 3.8%, or 4%.

In one embodiment of the present disclosure, the mass ratio of the active ingredient A to the active ingredient B may be 1:100 to 100:1, preferably 1:10 to 10:1, for example, 1:2, 1:1.7, 1:1.3, 1:1.1, or 1:1.

In one embodiment of the present invention, the active ingredient A is present in the pharmaceutical composition B in a mass percentage of 0.1% to 10%, preferably 0.5% to 5%, for example, 1%, 1.2%, 1.5%, 1.8%, or 2%.

In one embodiment of the present disclosure, the active ingredient B is present in the pharmaceutical composition B in a mass percentage of 0.1% to 10%, preferably 0.5% to 5%, for example, 2%.

In one embodiment of the present disclosure, the pharmaceutically acceptable carrier comprises a vehicle.

In one embodiment of the present disclosure, the pharmaceutically acceptable carrier comprises a transdermal penetration enhancer.

In one embodiment of the present disclosure, the pharmaceutically acceptable carrier may be a vehicle and a transdermal penetration enhancer.

In one embodiment of the present disclosure, the vehicle is one or more selected from water, a sulfoxide vehicle, and an alcohol vehicle; preferably, the vehicle is a mixed vehicle of a sulfoxide vehicle and an alcohol vehicle. The sulfoxide vehicle is preferably dimethyl sulfoxide. The alcohol vehicle is preferably one or more selected from propylene glycol, ethanol, glycerol, and polyethylene glycol 400, more preferably one or more selected from propylene glycol, ethanol, and polyethylene glycol 400, further preferably selected from propylene glycol and/or polyethylene glycol 400.

In one embodiment of the present disclosure, the vehicle is one or more selected from water, dimethyl sulfoxide, propylene glycol, ethanol, glycerol, and polyethylene glycol 400, preferably one or more selected from dimethyl sulfoxide, polyethylene glycol 400, propylene glycol, and ethanol, more preferably is a mixed vehicle of dimethyl sulfoxide, propylene glycol, and polyethylene glycol 400, or a mixed vehicle of dimethyl sulfoxide and propylene glycol.

In one embodiment of the present disclosure, the vehicle comprises at least a sulfoxide vehicle, preferably dimethyl sulfoxide.

In one embodiment of the present disclosure, the vehicle is present in the pharmaceutical composition B in a mass percentage of 20% to 50%, preferably 30% to 50%, for example, 27%, 28%, 32%, 33%, 37%, 37.2%, 37.5%, 37.8%, 38%, 42%, 43%, or 48%.

In one embodiment of the present disclosure, when the vehicle is a mixed vehicle of an alcohol vehicle and a sulfoxide vehicle, and the alcohol vehicle is propylene glycol, then the mass ratio of the propylene glycol to the sulfoxide vehicle is 3:1 to 9:1; preferably 4:1 to 8:1, for example, 4.6:1, 5.6:1, 6.6:1, or 7.6:1.

In one embodiment of the present disclosure, when the vehicle is a mixed vehicle of a sulfoxide vehicle and an alcohol vehicle, and the alcohol vehicle is propylene glycol and polyethylene glycol 400, then the mass ratio of the propylene glycol to the sulfoxide vehicle is 10:1 to 1:1, preferably 7:1 to 4:1, for example, 6.4:1 or 5.4:1; the mass ratio of the polyethylene glycol 400 to the sulfoxide vehicle is 0.5:1 to 5:1, preferably from 1:1 to 3:1, for example, 1:1 or 2:1.

In one embodiment of the present disclosure, the transdermal penetration enhancer is one or more selected from an ester reagent, a surfactant, an ether reagent, a bile salt reagent, an alcohol reagent, a terpenoid reagent, a sulfoxide reagent, and a cyclodextrin reagent. The ether reagent is preferably one or more selected from diethylene glycol monoethyl ether, polyoxyethylene lauryl ether, and polyethylene glycol cetyl ether. The bile salt reagent is preferably selected from deoxycholate and/or glycocholate. The ester reagent is preferably one or more selected from propylene glycol monolaurate, glyceryl mono and dicaprylocaprate, monocaprylin, ethyl oleate, isopropyl myristate, glyceryl monocaprylocaprate, propylene glycol monocaprylate, polyglycerol oleate, D-α-tocopheryl polyethylene glycol succinate, and soybean phospholipid. The surfactant is preferably one or more selected from Tween 20, Tween 40, Tween 60, Tween 80, Span 20, Span 40, Span 60, Span 80, Span 85, sodium dodecyl sulfate, polyoxyethylene (35) castor oil, and polyoxyethylene 40 hydrogenated castor oil. The alcohol reagent is preferably one or more selected from an aliphatic alcohol reagents and a polyol reagent; the aliphatic alcohol reagent is preferably one or more selected from ethanol, oleyl alcohol, and octyl dodecanol; the polyol reagent is preferably propylene glycol. The terpenoid reagent is preferably menthol. The sulfoxide reagent is preferably dimethyl sulfoxide. The cyclodextrin reagent is preferably hydroxypropyl-β-cyclodextrin.

In one embodiment of the present disclosure, the transdermal penetration enhancer is one or more selected from diethylene glycol monoethyl ether, hydroxypropyl-β-cyclodextrin, Tween 20, Tween 40, Tween 60, Tween 80, Span 20, Span 40, Span 60, Span 80, Span 85, deoxycholate, glycocholate, propylene glycol monolaurate, glyceryl mono and dicaprylocaprate, monocaprylin, ethyl oleate, isopropyl myristate, glyceryl monocaprylocaprate, propylene glycol monocaprylate, sodium dodecyl sulfate, polyoxyethylene lauryl ether, polyethylene glycol cetyl ether, menthol, polyglycerol oleate, polyoxyethylene (35) castor oil, oleyl alcohol, octyl dodecanol, polyoxyethylene 40 hydrogenated castor oil, D-α-tocopheryl polyethylene glycol succinate, and soybean phospholipid.

In one embodiment of the present disclosure, the transdermal penetration enhancer is preferably one or more selected from diethylene glycol monoethyl ether, hydroxypropyl-β-cyclodextrin, Tween 80, propylene glycol monolaurate, glyceryl mono and dicaprylocaprate, monocaprylin, ethyl oleate, glyceryl monocaprylocaprate, and propylene glycol monocaprylate, for example, a mixed transdermal penetration enhancer of diethylene glycol monoethyl ether and hydroxypropyl-β-cyclodextrin; a mixed transdermal penetration enhancer of diethylene glycol monoethyl ether, Tween 80, and propylene glycol monolaurate; a mixed transdermal penetration enhancer of diethylene glycol monoethyl ether and glyceryl mono and dicaprylocaprate; a mixed transdermal penetration enhancer of diethylene glycol monoethyl ether and glyceryl monocaprylocaprate; a mixed transdermal penetration enhancer of diethylene glycol monoethyl ether, Tween 80, and monocaprylin; a mixed transdermal penetration enhancer of diethylene glycol monoethyl ether and monocaprylin; a mixed transdermal penetration enhancer of diethylene glycol monoethyl ether and ethyl oleate; a mixed transdermal penetration enhancer of diethylene glycol monoethyl ether and propylene glycol monolaurate; a mixed transdermal penetration enhancer of diethylene glycol monoethyl ether, glyceryl mono and dicaprylocaprate, and glyceryl monocaprylocaprate; a mixed transdermal penetration enhancer of diethylene glycol monoethyl ether and propylene glycol monocaprylate; a mixed transdermal penetration enhancer of diethylene glycol monoethyl ether, monocaprylin, and glyceryl monocaprylocaprate; a mixed transdermal penetration enhancer of diethylene glycol monoethyl ether, glyceryl mono and dicaprylocaprate, and monocaprylin; or a mixed transdermal penetration enhancer of diethylene glycol monoethyl ether, monocaprylin, and propylene glycol monocaprylate.

In one embodiment of the present disclosure, the transdermal penetration enhancer is more preferably a mixed transdermal penetration enhancer of diethylene glycol monoethyl ether and propylene glycol monocaprylate, or a mixed transdermal penetration enhancer of diethylene glycol monoethyl ether, monocaprylin, and propylene glycol monocaprylate.

In one embodiment of the present disclosure, the transdermal penetration enhancer is present in the pharmaceutical composition B in a mass percentage of 40% to 80%, preferably 50% to 70%, for example, 49%, 54%, 59%, 64%, or 69%.

In one embodiment of the present disclosure, when the transdermal penetration enhancer is a mixed transdermal penetration enhancer of diethylene glycol monoethyl ether and hydroxypropyl-β-cyclodextrin, then the mass ratio of diethylene glycol monoethyl ether to hydroxypropyl-β-cyclodextrin is 2:1 to 4:1, for example, 3.27:1.

In one embodiment of the present disclosure, when the transdermal penetration enhancer is a mixed transdermal penetration enhancer of diethylene glycol monoethyl ether, Tween 80, and propylene glycol monolaurate, then the mass ratio of diethylene glycol monoethyl ether to propylene glycol monolaurate is 8:1 to 10:1, for example, 9.8:1. The mass ratio of Tween 80 to propylene glycol monolaurate is 2:1 to 4:1, for example, 3:1.

In one embodiment of the present disclosure, when the transdermal penetration enhancer is a mixed transdermal penetration enhancer of diethylene glycol monoethyl ether and glyceryl mono and dicaprylocaprate, then the mass ratio of diethylene glycol monoethyl ether to glyceryl mono and dicaprylocaprate is 4:1 to 10:1, for example, 4.9:1 or 9.8:1.

In one embodiment of the present disclosure, when the transdermal penetration enhancer is a mixed transdermal penetration enhancer of diethylene glycol monoethyl ether and glyceryl monocaprylocaprate, then the mass ratio of diethylene glycol monoethyl ether to glyceryl monocaprylocaprate is 8:1 to 10:1, for example, 9.8:1.

In one embodiment of the present disclosure, when the transdermal penetration enhancer is a mixed transdermal penetration enhancer of diethylene glycol monoethyl ether, Tween 80, and monocaprylin, then the mass ratio of diethylene glycol monoethyl ether to monocaprylin is 8:1 to 10:1, for example, 9.8:1. The mass ratio of Tween 80 to monocaprylin is 0.5:1 to 2:1, for example, 1:1.

In one embodiment of the present disclosure, when the transdermal penetration enhancer is a mixed transdermal penetration enhancer of diethylene glycol monoethyl ether and monocaprylin, then the mass ratio of diethylene glycol monoethyl ether to monocaprylin is 4:1 to 10:1, for example, 4.9:1 or 9.8:1.

In one embodiment of the present disclosure, when the transdermal penetration enhancer is a mixed transdermal penetration enhancer of diethylene glycol monoethyl ether and ethyl oleate, then the mass ratio of diethylene glycol monoethyl ether to ethyl oleate is 8:1 to 10:1, for example, 9.8:1.

In one embodiment of the present disclosure, when the transdermal penetration enhancer is a mixed transdermal penetration enhancer of diethylene glycol monoethyl ether and propylene glycol monolaurate, then the mass ratio of diethylene glycol monoethyl ether to propylene glycol monolaurate is 8:1 to 10:1, for example, 9.8:1.

In one embodiment of the present disclosure, when the transdermal penetration enhancer is a mixed transdermal penetration enhancer of diethylene glycol monoethyl ether, glyceryl mono and dicaprylocaprate, and glyceryl monocaprylocaprate, then the mass ratio of diethylene glycol monoethyl ether to glyceryl monocaprylocaprate is 8:1 to 10:1, for example, 9.8:1. The mass ratio of glyceryl mono and dicaprylocaprate to glyceryl monocaprylocaprate is 0.5:1 to 2:1, for example, 1:1.

In one embodiment of the present disclosure, when the transdermal penetration enhancer is a mixed transdermal penetration enhancer of diethylene glycol monoethyl ether and propylene glycol monocaprylate, then the mass ratio of diethylene glycol monoethyl ether to propylene glycol monocaprylate is 3:1 to 6:1, for example, 4.9:1.

In one embodiment of the present disclosure, when the transdermal penetration enhancer is a mixed transdermal penetration enhancer of diethylene glycol monoethyl ether, monocaprylin, and glyceryl monocaprylocaprate, then the mass ratio of diethylene glycol monoethyl ether to glyceryl monocaprylocaprate is 8:1 to 10:1, for example, 9.8:1. The mass ratio of monocaprylin to glyceryl monocaprylocaprate is 0.5:1 to 2:1, for example, 1:1.

In one embodiment of the present disclosure, when the transdermal penetration enhancer is a mixed transdermal penetration enhancer of diethylene glycol monoethyl ether, glyceryl mono and dicaprylocaprate, and monocaprylin, then the mass ratio of diethylene glycol monoethyl ether to monocaprylin is 8:1 to 10:1, for example, 9.8:1. The mass ratio of glyceryl mono and dicaprylocaprate to monocaprylin is 0.5:1 to 2:1, for example, 1:1.

In one embodiment of the present disclosure, when the transdermal penetration enhancer is a mixed transdermal penetration enhancer of diethylene glycol monoethyl ether, monocaprylin, and propylene glycol monocaprylate, then the mass ratio of diethylene glycol monoethyl ether to propylene glycol monocaprylate is 8:1 to 10:1, for example, 9.8:1. The mass ratio of monocaprylin to propylene glycol monocaprylate is 0.5:1 to 2:1, such as 1:1.

In one embodiment of the present disclosure, the pharmaceutical composition B consists of the active ingredient A, the active ingredient B, the vehicle, and the transdermal penetration enhancer.

In one embodiment of the present disclosure, the pharmaceutical composition B preferably consists of components as shown in any of the following schemes:
scheme one: 1% of the compound of formula I, 2% of minoxidil, 49% of diethylene glycol monoethyl ether, 28% of propylene glycol, 5% of dimethyl sulfoxide, and 15% of hydroxypropyl-β-cyclodextrin;
scheme two: 1% of the compound of formula I, 2% of minoxidil, 49% of diethylene glycol monoethyl ether, 23% of propylene glycol, 5% of dimethyl sulfoxide, 15% of Tween 80, and 5% of propylene glycol monolaurate;
scheme three: 1% of the compound of formula I, 2% of minoxidil, 49% of diethylene glycol monoethyl ether, 38% of propylene glycol, 5% of dimethyl sulfoxide, and 5% of glyceryl mono and dicaprylocaprate;
scheme four: 1% of the compound of formula I, 2% of minoxidil, 49% of diethylene glycol monoethyl ether, 38% of propylene glycol, 5% of dimethyl sulfoxide, and 5% of glyceryl monocaprylocaprate;
scheme five: 1% of the compound of formula I, 2% of minoxidil, 49% of diethylene glycol monoethyl ether, 33% of propylene glycol, 5% of dimethyl sulfoxide, 5% of Tween 80, and 5% of monocaprylin;
scheme six: 1% of the compound of formula I, 2% of minoxidil, 49% of diethylene glycol monoethyl ether, 38% of propylene glycol, 5% of dimethyl sulfoxide, and 5% of monocaprylin;
scheme seven: 1% of the compound of formula I, 2% of minoxidil, 49% of diethylene glycol monoethyl ether, 38% of propylene glycol, 5% of dimethyl sulfoxide, and 5% of ethyl oleate;
scheme eight: 1% of the compound of formula I, 2% of minoxidil, 49% of diethylene glycol monoethyl ether, 38% of propylene glycol, 5% of dimethyl sulfoxide, and 5% of propylene glycol monolaurate;
scheme nine: 1% of the compound of formula I, 2% of minoxidil, 49% of diethylene glycol monoethyl ether, 33% of propylene glycol, 5% of dimethyl sulfoxide, and 10% of glyceryl mono and dicaprylocaprate;
scheme ten: 1% of the compound of formula I, 2% of minoxidil, 49% of diethylene glycol monoethyl ether, 33% of propylene glycol, 5% of dimethyl sulfoxide, 5% of glyceryl mono and dicaprylocaprate, and 5% of glyceryl monocaprylocaprate;
scheme eleven: 1% of the compound of formula I, 2% of minoxidil, 49% of diethylene glycol monoethyl ether, 33% of propylene glycol, 5% of dimethyl sulfoxide, and 10% of propylene glycol monocaprylate;
scheme twelve: 1% of the compound of formula I, 2% of minoxidil, 49% of diethylene glycol monoethyl ether, 33% of propylene glycol, 5% of dimethyl sulfoxide, and 10% of monocaprylin;
scheme thirteen: 1% of the compound of formula I, 2% of minoxidil, 49% of diethylene glycol monoethyl ether, 33% of propylene glycol, 5% of dimethyl sulfoxide, 5% of monocaprylin, and 5% of glyceryl monocaprylocaprate;
scheme fourteen: 1% of the compound of formula I, 2% of minoxidil, 49% of diethylene glycol monoethyl ether, 33% of propylene glycol, 5% of dimethyl sulfoxide, 5% of glyceryl mono and dicaprylocaprate, and 5% of monocaprylin;
scheme fifteen: 1% of the compound of formula I, 2% of minoxidil, 49% of diethylene glycol monoethyl ether, 33% of propylene glycol, 5% of dimethyl sulfoxide, 5% of monocaprylin, and 5% of propylene glycol monocaprylate.

All the above percentages are mass percentages.

In one embodiment of the present disclosure, the pharmaceutical composition B preferably consists of 1% of the compound of formula I, 2% of minoxidil, 49% of diethylene glycol monoethyl ether, 33% of propylene glycol, 5% of dimethyl sulfoxide, 0 to 5% of monocaprylin, and 5 to 10% of propylene glycol monocaprylate. (The above percentages are mass percentages.)

In one embodiment of the present disclosure, the pharmaceutical composition B more preferably follows scheme eleven or scheme fifteen.

In one embodiment of the present disclosure, the dosage form of the pharmaceutical composition B may be a paste, a paint, a coating agent, a liniment, an emulsion, an ointment, a cream, a tincture, a patch, a plaster, or a gel; preferably a liniment.

The present disclosure further provides a preparation method for the aforementioned pharmaceutical composition B, said method comprising the following step: mixing the active ingredient A, the active ingredient B, and a pharmaceutically acceptable carrier, wherein the pharmaceutically acceptable carrier comprises a vehicle and a transdermal penetration enhancer.

In one embodiment of the present disclosure, when the vehicle comprises a sulfoxide vehicle, then the preparation method for the aforementioned pharmaceutical composition B preferably comprises the following steps:
(1) mixing the active ingredient A, the active ingredient B, and the sulfoxide vehicle to obtain a mixture 1;
(2) mixing the mixture 1 from step (1) with the other vehicle and the transdermal penetration enhancer to obtain the pharmaceutical composition B. Preferably, the active ingredient A is preferably a compound of formula I, and/or the active ingredient B is preferably minoxidil.

In one embodiment of the present disclosure, in step (2), said mixing may be performed by adding the other vehicle and the transdermal penetration enhancer to the mixture 1 from step (1).

In one embodiment of the present disclosure, in step (2), said mixing may be performed by stirring to achieve uniformity.

The present disclosure further provides a use of a compound of formula I, a pharmaceutically acceptable salt thereof, a solvate thereof, or a solvate of the pharmaceutically acceptable salt thereof, the combined drug as described above, the pharmaceutical composition A as described above, the pharmaceutical composition B as described above, or the combined drug kit as described above, in the manufacture of a medicament for preventing, alleviating, or treating alopecia areata, wherein said use is not a use of a pharmaceutical composition C in the manufacture of a medicament for preventing, alleviating, or treating alopecia areata, said pharmaceutical composition C comprising the following components by mass fraction: 0.1% to 3% of the compound of formula I or a pharmaceutically acceptable salt thereof, 35% to 93% of a vehicle, and 5% to 63% of a transdermal penetration enhancer; the vehicle is one or more selected from water, glycerol, polyethylene glycol 400, dimethyl sulfoxide, ethyl acetate, propylene glycol, and ethanol; the transdermal penetration enhancer is one or more selected from diethylene glycol monoethyl ether, azone, urea, oleic acid, diisopropyl adipate, menthol, N-methylpyrrolidone, imidurea, propyl gallate, isopropyl myristate, cyclodextrin, sodium dodecyl sulfate, polyoxyethylene lauryl ether, polyethylene glycol cetyl ether, Tween 20, Tween 40, Tween 60, Tween 80, Span 20, Span 40, Span 60, Span 80, Span 85, deoxycholate, glycocholate, monocaprylin, glyceryl mono and dicaprylocaprate, propylene glycol monolaurate, propylene glycol monocaprylate, and glyceryl behenate.

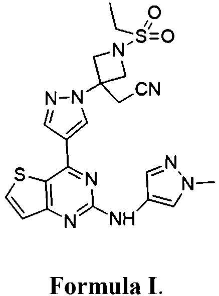

The present disclosure further provides a method for treating alopecia areata, comprising administering to a subject (e.g., a human) in need thereof a therapeutically effective amount of the compound of formula I as described above, a pharmaceutically acceptable salt thereof, a solvate thereof, or a solvate of the pharmaceutically acceptable salt thereof, the combined drug as described above; the pharmaceutical composition A as described above; the pharmaceutical composition B as described above; or the combined drug kit as described above, wherein the method does not comprise administering to the subject in need thereof (e.g., a human) a therapeutically effective amount of a pharmaceutical composition C, said pharmaceutical composition C comprising the following components in mass fractions: 0.1% to 3% of the compound of formula I or a pharmaceutically acceptable salt thereof, 35% to 93% of a vehicle, and 5% to 63% of a transdermal penetration enhancer; the vehicle is one or more selected from water, glycerol, polyethylene glycol 400, dimethyl sulfoxide, ethyl acetate, propylene glycol, and ethanol; the transdermal penetration enhancer is one or more selected from diethylene glycol monoethyl ether, azone, urea, oleic acid, diisopropyl adipate, menthol, N-methylpyrrolidone, imidurea, propyl gallate, isopropyl myristate, cyclodextrin, sodium dodecyl sulfate, polyoxyethylene lauryl ether, polyethylene glycol cetyl ether, Tween 20, Tween 40, Tween 60, Tween 80, Span 20, Span 40, Span 60, Span 80, Span 85, deoxycholate, glycocholate, monocaprylin, glyceryl mono and dicaprylocaprate, propylene glycol monolaurate, propylene glycol monocaprylate, and glyceryl behenate.

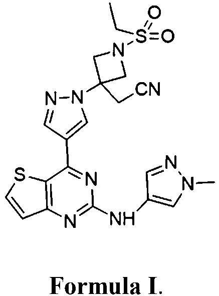

In addition to the foregoing, unless otherwise specified, the following terms have the meanings shown below when used in the description and claims of the present disclosure:
The term "pharmaceutically acceptable salt" refers to a salt of a compound of the present invention prepared from a relatively non-toxic, pharmaceutically acceptable acid or base. When the compounds of the present invention contain relatively acidic functional groups, base addition salts can be obtained by contacting the neutral form of such compounds with a sufficient amount of a pharmaceutically acceptable base in a pure solution or a suitable inert vehicle. When the compound of the present invention contains a relatively basic functional group, an acid addition salt can be obtained by contacting the neutral form of such a compound with a sufficient amount of a pharmaceutically acceptable acid in a pure solution or a suitable inert vehicle.

The term "solvate" refers to a substance formed by the crystallization of a compound with a vehicle (including, but not limited to: water, methanol, ethanol, etc.). Solvates are classified into stoichiometric solvates and non-stoichiometric solvates.

The term "pharmaceutically acceptable salt solvate" refers to a substance formed by the combination of a compound with a pharmaceutically acceptable (relatively non-toxic, safe, and suitable for patient use) acid or base, and a vehicle (including but not limited to: water, methanol, ethanol, etc.), wherein the pharmaceutically acceptable salt has the same meaning as the term "pharmaceutically acceptable salt" above, and the vehicle may be stoichiometric or non-stoichiometric.

The term "pharmaceutically acceptable carrier" refers to excipients and additives used in the production of pharmaceuticals and the preparation of formulations. It encompasses all substances included in a drug formulation other than the active ingredient. Please refer to the Pharmacopoeia of the People's Republic of China (2015 Edition) Part IV or the Handbook of Pharmaceutical Excipients (Raymond C Rowe, 2009 Sixth Edition).

The term "transdermal penetration enhancer" is also referred to as "transdermal absorption enhancer," which denotes a material capable of penetrating into the skin to reduce the resistance of drugs passing through the skin. It can assist drugs in crossing the stratum corneum, diffusing through the skin, and entering the systemic circulation via capillaries. A transdermal penetration enhancer should meet the following conditions: 1) It should have no pharmacological effect on the skin and body, be non-toxic, non-irritating, and non-allergenic; 2) It should take effect immediately upon application, and the skin should be able to restore its normal barrier function after removal; 3) It should not cause the loss of nutrients and moisture from the body through the skin; 4) It should not interact physically or chemically with the drug or other excipients.

Without departing from the common knowledge in the art, the above preferred conditions may be combined arbitrarily to obtain various preferred embodiments of the present invention.

The reagents and raw materials used in the present invention are all commercially available.

The positive and progressive effects of the present disclosure lie in that: the combined drug and the pharmaceutical composition of the present disclosure have good permeability, stability and safety, have a good therapeutic effect on alopecia areata, and are suitable for topical administration.

### BRIEF DESCRIPTION OF THE DRAWINGS

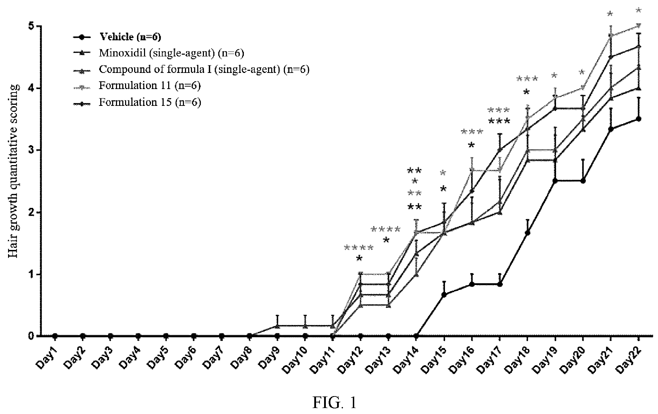

FIG.1 shows the change curve of hair growth scores in each group of mice.

### DETAILED DESCRIPTION OF THE PREFERRED EMBODIMENT

The following further illustrates the present disclosure by way of examples, but does not thereby limit the present disclosure to the scope of the described examples. In the following examples, the experimental methods without specified conditions are carried out in accordance with conventional methods and conditions, or according to the instructions of commercial products.

Diethylene glycol monoethyl ether (Transcutol P); hydroxypropyl-β-cyclodextrin (HP-β-CD); propylene glycol monolaurate (Lauroglycol FCC); glyceryl mono and dicaprylocaprate (Capmul MCM); monocaprylin (Capmul MCM-C8); glyceryl monocaprylocaprate (Labrasol); propylene glycol monocaprylate (Capmul PG-8); polyoxyethylene (35) castor oil (Kolliphor ELP); oleyl alcohol (Kollicream OA); octyl dodecanol (Kollicream OD); polyoxyethylene 40 hydrogenated castor oil (Kolliphor RH40); polyglycerol oleate (PLUROL OLEIQUE CC497), D-α-tocopheryl polyethylene glycol succinate (VE TPGS NF (1000)), polyoxyethylene lauryl ether (Brij L23), polyethylene glycol cetyl ether (Brij C10); compound of formula I 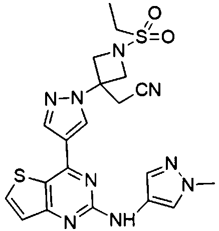 : produced by Guangzhou MaxiNovel Pharmaceuticals Co.,Ltd.; minoxidil API: purchased from Tianjin Institute of Pharmaceutical Research Pharmaceutical Co., Ltd., batch number: 2011052.

### Example 1: Formulation and preparation method of pharmaceutical composition

**Table 1-1: Components (w/w%) in formulations of pharmaceutical compositions**

| **Component/Pharmaceutical composition** | **Formulation 1** | **Formulation 2** | **Formulation 3** | **Formulation 4** | **Formulation 5** | **Formulation 6** | **Formulation 7** | **Formulation 8** | **Formulation 9** |
|---|---|---|---|---|---|---|---|---|---|
| Compound of formula I | 1% | 1% | 1% | 1% | 1% | 1% | 1% | 1% | 1% |
| Minoxidil API | 2% | 2% | 2% | 2% | 2% | 2% | 2% | 2% | 2% |
| Transcutol P | 49% | 49% | 49% | 49% | 49% | 49% | 49% | 49% | 49% |
| Propylene glycol | 28% | 23% | 38% | 38% | 33% | 38% | 38% | 38% | 33% |
| DMSO | 5% | 5% | 5% | 5% | 5% | 5% | 5% | 5% | 5% |
| HP-β-CD | 15%- | -- | -- | -- | -- | -- | -- | -- | -- |
| Tween 80 | -- | 15% | -- | -- | 5% | -- | -- | -- | -- |
| Lauroglycol FCC | -- | 5% | -- | -- | -- | -- | -- | 5% | -- |
| Capmul MCM | -- | -- | 5% | -- | -- | -- | -- | -- | 10% |
| Capmul MCM-C8 | -- | -- | -- | -- | 5% | 5% | -- | -- | -- |
| Ethyl oleate | -- | -- | -- | -- | -- | -- | 5% | -- | -- |
| Labrasol | -- | -- | -- | 5% | -- | -- | -- | -- | -- |
| Capmul PG-8 | -- | -- | -- | -- | -- | -- | -- | -- | -- |
| Total | 100% | 100% | 100% | 100% | 100% | 100% | 100% | 100% | 100% |

**Table 1-2: Components (w/w%) in formulations of pharmaceutical compositions**

| **Component/Pharmaceutical composition** | **Formulation 10** | **Formulation 11** | **Formulation 12** | **Formulation 13** | **Formulation 14** | **Formulation 15** | **Formulation 16** |
|---|---|---|---|---|---|---|---|
| Compound of formula I | 1% | 1% | 1% | 1% | 1% | 1% | 1% |
| Minoxidil API | 2% | 2% | 2% | 2% | 2% | 2% | 2% |
| Transcutol P | 49% | 49% | 49% | 49% | 49% | 49% | 49% |
| Propylene glycol | 33% | 33% | 33% | 33% | 33% | 33% | 43% |
| DMSO | 5% | 5% | 5% | 5% | 5% | 5% | 5% |
| HP-β-CD | -- | -- | -- | -- | -- | -- | -- |
| Tween 80 | -- | -- | -- | -- | -- | -- | -- |
| Lauroglycol FCC | -- | -- | -- | -- | -- | -- | -- |
| Capmul MCM | 5% | -- | -- | -- | 5% | -- | -- |
| Capmul MCM-C8 | -- | -- | 10% | 5% | 5% | 5% | -- |
| Ethyl oleate | -- | -- | -- | -- | -- | -- | -- |
| Labrasol | 5% | -- | -- | 5% | -- | -- | -- |
| Capmul PG-8 | -- | 10% | -- | -- | -- | 5% | -- |
| Total | 100% | 100% | 100% | 100% | 100% | 100% | 100% |

| | | | | | | | |
|---|---|---|---|---|---|---|---|
| Note: The percentages in the above table are all mass percentages. | | | | | | | |

The results indicated that among samples of the above pharmaceutical composition formulations stored at room temperature for 3 months, oil droplets were observed to precipitate in pharmaceutical composition formulation 7, solid precipitates were observed to precipitate in pharmaceutical composition formulation 10, and the other pharmaceutical composition formulations were relatively stable.

**Table 2-1: Components (w/w%) in formulations of pharmaceutical compositions**

| **Component/P harmaceutical composition** | **Formulation 17** | **Formulation 18** | **Formulation 19** | **Formulation 20** | **Formulation 21** | **Formulation 22** | **Formulation 23** | **Formulation 24** |
|---|---|---|---|---|---|---|---|---|
| Compound of formula I | 2% | 2% | 2% | 2% | 2% | 2% | 2% | 2% |
| Minoxidil API | 2% | 2% | 2% | 2% | 2% | 2% | 2% | 2% |
| Transcutol P | 49% | 49% | 49% | 49% | 49% | 49% | 49% | 49% |
| Propylene glycol | 37% | 37% | 37% | 37% | 37% | 37% | 37% | 37% |
| DMSO | 5% | 5% | 5% | 5% | 5% | 5% | 5% | 5% |
| Labrasol | 5% | -- | -- | -- | -- | -- | -- | -- |
| Lauroglycol FCC | -- | 5% | -- | -- | -- | -- | -- | -- |
| Capmu PG-8 | -- | -- | 5% | -- | -- | -- | -- | -- |
| Capmu MCM-C8 | -- | -- | -- | 5% | -- | -- | -- | -- |
| Tween 80 | -- | -- | -- | -- | 5% | -- | -- | -- |
| Tween 60 | -- | -- | -- | -- | -- | 5% | -- | -- |
| Polyglycerol oleate | -- | -- | -- | -- | -- | -- | 5% | -- |
| Kolliphor ELP | -- | -- | -- | -- | -- | -- | -- | 5% |
| Kollicream OA | -- | -- | -- | -- | -- | -- | -- | -- |
| Kollicream OD | -- | -- | -- | -- | -- | -- | -- | -- |
| Capmul MCM | -- | -- | -- | -- | -- | -- | -- | -- |
| Kolliphor RH40 | -- | -- | -- | -- | -- | -- | -- | -- |
| VeTPGS | -- | -- | -- | -- | -- | -- | -- | -- |
| Soybean phospholipid | -- | -- | -- | -- | -- | -- | -- | -- |
| HP-β-CD | -- | -- | -- | -- | -- | -- | -- | -- |
| Total | 100% | 100% | 100% | 100% | 100% | 100% | 100% | 100% |

**Table 2-2: Components (w/w%) in formulations of pharmaceutical compositions**

| **Component/Pharmaceutical composition** | **Formulation 25** | **Formulation 26** | **Formulation 27** | **Formulation 28** | **Formulation 29** | **Formulation 30** | **Formulation 31** |
|---|---|---|---|---|---|---|---|
| Compound of formula I | 2% | 2% | 2% | 2% | 2% | 2% | 2% |
| Minoxidil API | 2% | 2% | 2% | 2% | 2% | 2% | 2% |
| Transcutol P | 49% | 49% | 49% | 49% | 49% | 49% | 49% |
| Propylene glycol | 37% | 37% | 37% | 37% | 37% | 37% | 37% |
| DMSO | 5% | 5% | 5% | 5% | 5% | 5% | 5% |
| Labrasol | -- | -- | -- | -- | -- | -- | -- |
| Lauroglycol FCC | -- | -- | -- | -- | -- | -- | -- |
| Capmu PG-8 | -- | -- | -- | -- | -- | -- | -- |
| Capmu MCM-C8 | -- | -- | -- | -- | -- | -- | -- |
| Tween 80 | -- | -- | -- | -- | -- | -- | -- |
| Tween 60 | -- | -- | -- | -- | -- | -- | -- |
| Polyglycerol oleate | -- | -- | -- | -- | -- | -- | -- |
| Kolliphor ELP | -- | -- | -- | -- | -- | -- | -- |
| Kollicream OA | 5% | -- | -- | -- | -- | -- | -- |
| Kollicream OD | -- | 5% | -- | -- | -- | -- | -- |
| Capmul MCM | -- | -- | 5% | -- | -- | -- | -- |
| Kolliphor RH40 | -- | -- | -- | 5% | -- | -- | -- |
| VeTPGS | -- | -- | -- | -- | 5% | -- | -- |
| Soybean phospholipid | -- | -- | -- | -- | -- | 5% | -- |
| HP-β-CD | -- | -- | -- | -- | -- | -- | 5% |
| Total | 100% | 100% | 100% | 100% | 100% | 100% | 100% |

| | | | | | | | |
|---|---|---|---|---|---|---|---|
| Note: The percentages in the above table are all mass percentages. | | | | | | | |

**Table 3-1: Components (w/w%) in formulations of pharmaceutical compositions**

| **Component/ Pharmaceutical composition** | **Formulation 32** | **Formulation 33** | **Formulation 34** | **Formulation 35** | **Formulation 36** | **Formulation 37** | **Formulation 38** | **Formulation 39** | **Formulation 40** |
|---|---|---|---|---|---|---|---|---|---|
| Compound of formula I | 2% | 2% | 2% | 2% | 2% | 2% | 2% | 1.20 % | 1.80 % |
| Minoxidil API | 2% | 2% | 2% | 2% | 2% | 2% | 2% | 2% | 2% |
| Transcutol P | 49% | 49% | 49% | 49% | 49% | 49% | 49% | 49% | 49% |
| Propylene glycol | 32% | 22% | 32% | 27% | 32% | 32% | 32% | 32.80 % | 32.20 % |
| DMSO | 5% | 5% | 5% | 5% | 5% | 5% | 5% | 5% | 5% |
| Tween 80 | 5% | 15% | -- | -- | 5% | 5% | 5% | -- | -- |
| Lauroglycol FCC | 5% | 5% | 5% | 5% | -- | -- | -- | -- | -- |
| PEG400 | -- | -- | 5% | 10% | -- | -- | -- | -- | -- |
| Capmul MCM | -- | -- | -- | -- | -- | -- | -- | -- | -- |
| Capmul PG-8 | -- | -- | -- | -- | -- | -- | -- | 10% | 10% |
| Capmul MCM-C8 | -- | -- | -- | -- | 5% | -- | -- | -- | -- |
| Kollicream OD | -- | -- | -- | -- | -- | 5% | -- | -- | -- |
| Kolliphor RH40 | -- | -- | -- | -- | -- | -- | 5% | -- | -- |
| Total | 100% | 100% | 100% | 100% | 100% | 100% | 100% | 100% | 100% |

**Table 3-2: Components (w/w%) in formulations of pharmaceutical compositions**

| **Component/ Pharmaceutical composition** | **Formulation 41** | **Formulation 42** | **Formulation 43** | **Formulation 44** | **Formulation 45** | **Formulation 46** | **Formulation 47** |
|---|---|---|---|---|---|---|---|
| Compound of formula I | 1.50% | 2% | 1.50% | 2% | 1.50% | 2% | 1.5% |
| Minoxidil API | 2% | 2% | 2% | 2% | 2% | 2% | 2% |
| Transcutol P | 49% | 49% | 49% | 49% | 49% | 49% | 49% |
| Propylene glycol | 32.50 % | 32% | 32.50% | 32% | 32.50% | 32% | 32.5% |
| DMSO | 5% | 5% | 5% | 5% | 5% | 5% | 5% |
| Tween 80 | -- | -- | -- | -- | -- | -- | -- |
| Lauroglycol FCC | -- | -- | -- | -- | -- | -- | -- |
| PEG400 | -- | -- | -- | -- | -- | -- | -- |
| Capmul MCM | -- | -- | 10% | 10% | -- | -- | -- |
| Capmul PG-8 | 10% | 10% | --- | -- | 5% | 5% | -- |
| Capmul MCM-C8 | -- | -- | -- | -- | 5% | 5% | 10% |
| Kollicream OD | -- | -- | -- | -- | -- | -- | -- |
| Kolliphor RH40 | -- | -- | -- | -- | -- | -- | -- |
| Total | 100% | 100% | 100% | 100% | 100% | 100% | 100% |

| | | | | | | | |
|---|---|---|---|---|---|---|---|
| Note: The percentages in the above table are all mass percentages. | | | | | | | |

Preparation method: The compound of formula I and the minoxidil API were dissolved in dimethyl sulfoxide, followed by addition of other vehicles and a transdermal penetration enhancer, and stirred until uniform.

The formulations in Table 1-Table 3 were all prepared into corresponding pharmaceutical compositions according to the aforementioned preparation method.

### Example 2: In vitro transdermal penetration experiment

### (1) Pre-experiment preparation

Receiving liquid preparation: 400g of propylene glycol and 600g of purified water were weighed and mixed evenly in a beaker.

Skin preparation: Bama miniature pig skin was taken, with hair and fat removed, and set aside for use.

### (2) Experimental procedure

The transdermal diffusion instrument was turned on, with the temperature set at 32°C and the rotation speed at 200 rpm.

A diffusion cell was added with a stirrer and receiving solution, and placed on an instrument for preheating to 32°C.

The treated pig skin was added to the diffusion cell, and approximately 20 mg of the test sample (i.e., the pharmaceutical composition prepared according to the formulation in Example 1) was added.

After 24 hours, the receiving solution was collected, filtered, and the drug content was measured, recorded as the 24-hour cumulative penetration amount.

### (3) Post-experiment pig skin handling

The pig skin was removed and rinsed on the surface with a diluent (a mixed solution of 50% acetonitrile and 50% purified water). The pig skin from the administration site was cut into pieces, soaked in 10 mL of extraction solution (a mixed solution of 50% acetonitrile and 50% purified water) for 1 hour to extract the drug from the pig skin. After filtration, the drug content in the extraction solution was detected and recorded as the skin retention amount.

### (4) Experimental results

**Table 4**

| Pharmaceutical composition | Minoxidil | | Compound of formula I | |
|---|---|---|---|---|
| | Cumulative penetration amount over 24 hours (µg) | Skin retention amount (µg) | Cumulative penetration amount over 24 hours (µg) | Skin retention amount (µg) |
| Formulation 1 | 327.52 | 1.99 | 9.30 | 2.52 |
| Formulation 2 | 302.72 | 3.77 | 7.37 | 3.33 |
| Formulation 3 | 336.49 | 0.45 | 12.81 | 8.13 |
| Formulation 4 | 307.56 | 9.14 | 10.19 | 5.54 |
| Formulation 5 | 318.89 | 1.66 | 11.26 | 6.70 |
| Formulation 6 | 255.28 | 22.42 | 2.32 | 8.62 |
| Formulation 7 | 311.26 | 10.66 | 3.76 | 12.76 |
| Formulation 8 | 339.56 | 11.35 | 7.75 | 12.00 |
| Formulation 9 | 260.49 | 23.92 | 2.70 | 9.09 |
| Formulation 10 | 345.42 | 15.70 | 5.49 | 10.97 |
| Formulation 11 | 336.97 | 2.95 | 5.43 | 8.71 |
| Formulation 12 | 337.70 | 5.14 | 5.46 | 7.87 |
| Formulation 13 | 343.00 | 10.57 | 5.47 | 15.49 |
| Formulation 14 | 334.30 | 11.54 | 6.03 | 14.99 |
| Formulation 15 | 373.96 | 11.55 | 6.51 | 19.30 |
| Formulation 16 | 290.11 | 44.24 | 3.21 | 20.67 |
| Formulation 39 | 358.45 | 3.65 | 4.74 | 24.26 |
| Formulation 40 | 362.57 | 4.95 | 3.68 | 25.98 |
| Formulation 41 | 341.70 | 4.38 | 5.44 | 11.65 |
| Formulation 42 | 328.17 | 6.71 | 2.46 | 31.25 |
| Formulation 43 | 294.67 | 9.31 | 2.06 | 19.04 |
| Formulation 44 | 312.33 | 6.71 | 1.39 | 20.35 |
| Formulation 45 | 345.11 | 4.77 | 5.26 | 20.75 |
| Formulation 46 | 314.50 | 5.38 | 1.83 | 47.26 |
| Formulation 47 | 309.39 | 2.64 | 4.80 | 7.76 |

The results showed that, by comprehensively comparing the skin retention and cumulative penetration over 24 hours, pharmaceutical composition formulation 15 demonstrated relatively good penetration efficacy. Further combined with its stability performance, pharmaceutical composition formulation 15 was selected as the preferred formulation.

### Example 3: Preparation method for pharmaceutical composition

**Table 5**

| **Component/Pha rmaceutical composition** | **Formulation 11** | **Formulation 15** |
|---|---|---|
| Compound of formula I | 1 | 1 |
| Minoxidil API | 2 | 2 |
| Transcutol P | 49 | 49 |
| Propylene glycol | 33 | 33 |
| DMSO | 5 | 5 |
| Capmul PG-8 | 10 | 5 |
| Capmul MCM-C8 | 0 | 5 |
| Total | 100 | 100 |

According to the formulation composition in Table 5, the compound of formula I and the minoxidil API were dissolved in dimethyl sulfoxide, followed by sequential addition of Transcutol P, propylene glycol, Capmul PG-8 and/or Capmul MCM-C8, and mixed until uniform.

### Example 4: Efficacy experiment of pharmaceutical composition in treating alopecia areata

### (1) Experimental animals

Forty-nine-day-old female C57BL/6N mice, 60 in total, weighing 16 to 19 g, SPF grade, were acquired. Animals were screened based on the condition of their shaved backs, and those with pink, spot-free skin after shaving were chosen for the experiment. Zhejiang Vital River Laboratory Animal Technology Co., Ltd., SCXK (Zhe) 2019-0001, 20221117Abzz0619000331.

### (2) Formulation of pharmaceutical composition

**Table 6**

| **Component/Pha rmaceutical composition** | **Blank control group** | **Minoxid il (single-agent) liniment** | **Formulati on 11 liniment** | **Formulati on 15 liniment** | **Compound of formula I (single-agent) liniment** |
|---|---|---|---|---|---|
| Compound of formula I | 0 | 0 | 1 | 1 | 1 |
| Minoxidil | 0 | 2 | 2 | 2 | 0 |
| Transcutol P | 49 | 0 | 49 | 49 | 49 |
| Propylene glycol | 36 | 20 | 33 | 33 | 35 |
| DMSO | 5 | 0 | 5 | 5 | 5 |
| Capmul PG-8 | 5 | 0 | 10 | 5 | 5 |
| Capmul MCM-C8 | 5 | 0 | 0 | 5 | 5 |
| Ethanol | 0 | 60 | 0 | 0 | 0 |
| Water | 0 | 18 | 0 | 0 | 0 |
| Total | 100 | 100 | 100 | 100 | 100 |

### (3) Administration regimen

**Table 7**

| **Group** | **Numb er of mice** | **Formulati on name** | **Specificati ons** | **Administrat ion volume** | **Administrat ion frequency** | **Administrat ion site** |
|---|---|---|---|---|---|---|
| 1 | 6 | Blank control group | Blank | 20 µL per time | Twice daily | Upper back |
| 2 | 6 | Minoxidil (single-agent) liniment | 2% Minoxidil | 20 µL per time | Twice daily | Upper back |
| 3 | 6 | Formulatio n 11 liniment | 1% Compound of formula I 2% Minoxidil | 20 µL per time | Twice daily | Upper back |
| 4 | 6 | Formulatio n 15 liniment | 1% Compound of formula I 2% Minoxidil | 20 µL per time | Twice daily | Upper back |
| 5 | 6 | Compound of formula I (single-agent) liniment | 1% Compound of formula I | 20 µL per time | Twice daily | Upper back |

### (4) Experimental procedure

On the day before the start of administration, 30 animals with pink back skin, no black spots, and no damage were selected based on skin color and hair growth. The animals were randomly re-caged at a density of 3 animals per cage. A permanent marker was used to number the tails of the animals, and the numbers were recorded. Administration was performed twice daily. Starting from the second day of administration, photographs were taken and hair growth was scored daily between the two administrations. The body weight of the mice was measured and recorded daily.

### (5) Experimental results

### 5.1 Hair growth scoring grade assessment

0 points: The skin is pink and tender, consistent with the initial state after shaving.
1 point: The skin in the administration area turns gray to black, with no significant hair growth.
2 points: Short hair growth is locally observed in the administration area.
3 points: Short hair growth is observed in most of the administration areas, with sparse long hair present.
4 points: There is dense long hair growth, but it does not cover the entire administration area.
5 points: Dense long hair covers the entire administration area.
5.2 Hair growth score

Table 8

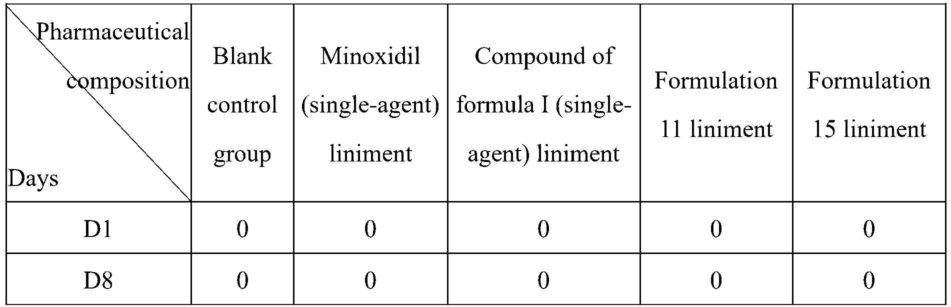

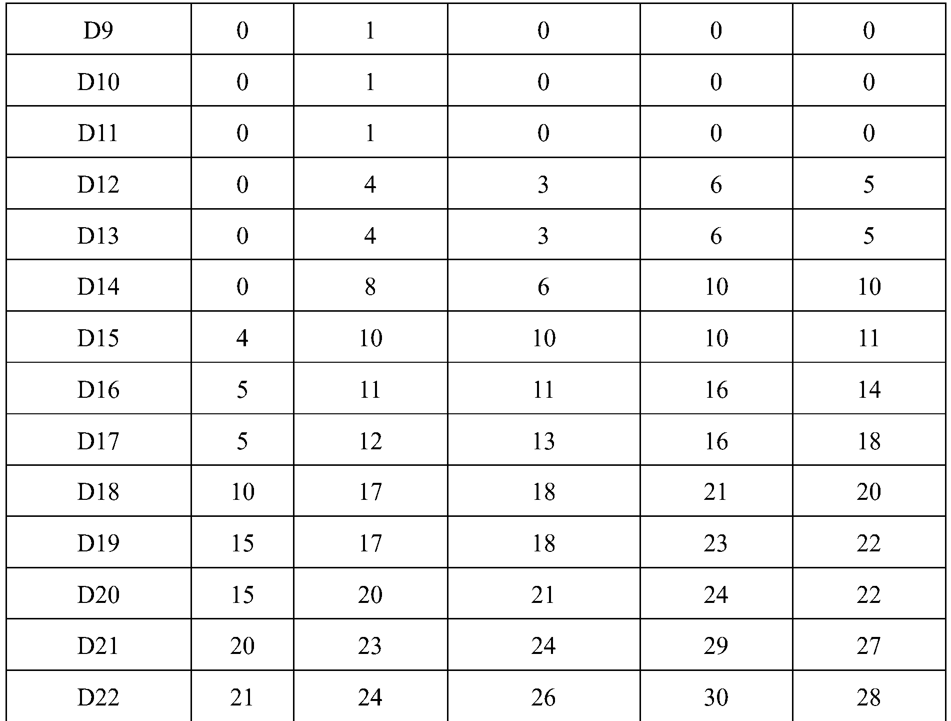

The results showed that compared with the blank control group, the hair growth scores of the formulation 11 liniment and the formulation 15 liniment began to show significant differences from the 12th day of transdermal administration; the therapeutic effects of the formulation 11 liniment and the formulation 15 liniment were both superior to those of the minoxidil (single-agent) liniment and the compound of formula I (single-agent) liniment (see Table 8 and FIG. 1). No significant abnormalities were observed in animal body weight during the concurrent administration period, indicating that formulation 11 liniment and formulation 15 liniment had no obvious toxicity or side effects.

## Claims

1. A combined drug, wherein active ingredients thereof comprise an active ingredient A and an active ingredient B;
wherein the active ingredient A is a compound of formula I, a pharmaceutically acceptable salt thereof, a solvate thereof, or a solvate of the pharmaceutically acceptable salt thereof;
the active ingredient B is minoxidil, a pharmaceutically acceptable salt thereof, a solvate thereof, or a solvate of the pharmaceutically acceptable salt thereof;

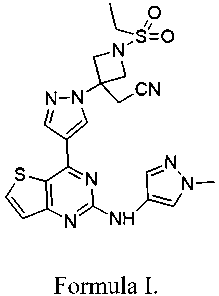

2. The combined drug according to claim 1, wherein the combined drug satisfies one or more of the following conditions:
(1) the mass ratio of the active ingredient A to the active ingredient B is 1:100 to 100:1, preferably 1:10 to 10:1, for example, 1:2, 1:1.7, 1:1.3, 1:1.1, or 1:1;
(2) the dosage form of the combined drug is a paste, a paint, a coating agent, a liniment, an emulsion, an ointment, a cream, a tincture, a patch, a plaster, or a gel; preferably a liniment;
(3) the combined drug is a combined drug for preventing, alleviating, or treating alopecia areata;
(4) the active ingredients consist of the active ingredient A and the active ingredient B;
(5) the active ingredient A and the active ingredient B are administered simultaneously or separately.

3. A pharmaceutical composition A, comprising a first pharmaceutical composition and a second pharmaceutical composition;
wherein the first pharmaceutical composition comprises an active ingredient A and a pharmaceutically acceptable carrier, and the active ingredient A is a compound of formula I, a pharmaceutically acceptable salt thereof, a solvate thereof, or a solvate of the pharmaceutically acceptable salt thereof;
the second pharmaceutical composition comprises an active ingredient B and a pharmaceutically acceptable carrier, and the active ingredient B is minoxidil, a pharmaceutically acceptable salt thereof, a solvate thereof, or a solvate of the pharmaceutically acceptable salt thereof;

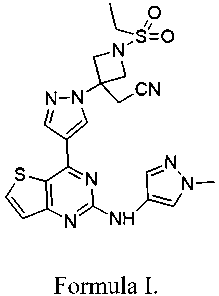

4. The pharmaceutical composition A according to claim 3, wherein the pharmaceutical composition A satisfies one or more of the following conditions:
(1) the mass ratio of the active ingredient A to the active ingredient B is 1:100 to 100:1, preferably 1:10 to 10:1, for example, 1:2, 1:1.7, 1:1.3, 1:1.1, or 1:1;
(2) the dosage form of the first pharmaceutical composition is a paste, a paint, a coating agent, a liniment, an emulsion, an ointment, a cream, a tincture, a patch, a plaster, or a gel, preferably a liniment;
(3) the dosage form of the second pharmaceutical composition is a paste, a paint, a coating agent, a liniment, an emulsion, an ointment, a cream, a tincture, a patch, a plaster, or a gel, preferably a liniment;
(4) the dosage form of the pharmaceutical composition A is a paste, a paint, a coating agent, a liniment, an emulsion, an ointment, a cream, a tincture, a patch, a plaster, or a gel, preferably a liniment;
(5) the pharmaceutical composition A is a pharmaceutical composition for preventing, alleviating, or treating alopecia areata;
(6) the pharmaceutical composition A consists of the first pharmaceutical composition and the second pharmaceutical composition;
(7) the first pharmaceutical composition consists of the active ingredient A and the pharmaceutically acceptable carrier;
(8) the second pharmaceutical composition consists of the active ingredient B and the pharmaceutically acceptable carrier;
(9) the first pharmaceutical composition and the second pharmaceutical composition are administered simultaneously or separately.

5. A pharmaceutical composition B, comprising active ingredients and a pharmaceutically acceptable carrier;
wherein the active ingredients comprise an active ingredient A and an active ingredient B;
the active ingredient A is a compound of formula I, a pharmaceutically acceptable salt thereof, a solvate thereof, or a solvate of the pharmaceutically acceptable salt thereof;
the active ingredient B is minoxidil, a pharmaceutically acceptable salt thereof, a solvate thereof, or a solvate of the pharmaceutically acceptable salt thereof;

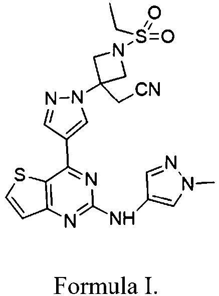

6. The pharmaceutical composition B according to claim 5, wherein the pharmaceutical composition B satisfies one or more of the following conditions:
(1) the pharmaceutical composition B is a pharmaceutical composition for preventing, alleviating, or treating alopecia areata;
(2) the active ingredients are present in the pharmaceutical composition B in a mass percentage of 0.2% to 20%, preferably 1% to 10%, for example, 3%, 3.2%, 3.5%, 3.8%, or 4%;
(3) the pharmaceutically acceptable carrier comprises a vehicle;
(4) the pharmaceutically acceptable carrier comprises a transdermal penetration enhancer;
(5) the dosage form of the pharmaceutical composition B is a paste, a paint, a coating agent, a liniment, an emulsion, an ointment, a cream, a tincture, a patch, a plaster, or a gel, preferably a liniment;
(6) the active ingredients consist of the active ingredient A and the active ingredient B.

7. The pharmaceutical composition B according to claim 6, wherein the pharmaceutical composition B satisfies one or more of the following conditions:
(1) the mass ratio of the active ingredient A to the active ingredient B is 1:100 to 100:1, preferably 1:10 to 10:1, for example, 1:2, 1:1.7, 1:1.3, 1:1.1, or 1:1;
(2) the active ingredient A is present in the pharmaceutical composition B in a mass percentage of 0.1% to 10%, preferably 0.5% to 5%, for example, 1%, 1.2%, 1.5%, 1.8%, or 2%;
(3) the active ingredient B is present in the pharmaceutical composition B in a mass percentage of 0.1% to 10%, preferably 0.5% to 5%, for example, 2%;
(4) the pharmaceutically acceptable carrier is a vehicle and a transdermal penetration enhancer;
(5) the vehicle is one or more selected from water, a sulfoxide vehicle, and an alcohol vehicle, preferably a mixed vehicle of a sulfoxide vehicle and an alcohol vehicle; the sulfoxide vehicle is preferably dimethyl sulfoxide; the alcohol vehicle is preferably one or more selected from propylene glycol, ethanol, glycerol, and polyethylene glycol 400, more preferably one or more selected from propylene glycol, ethanol, and polyethylene glycol 400, further preferably selected from propylene glycol and/or polyethylene glycol 400;
preferably, the vehicle comprises at least a sulfoxide vehicle, preferably dimethyl sulfoxide;
(6) the vehicle is present in the pharmaceutical composition B in a mass percentage of 20% to 50%, preferably 30% to 50%, for example, 27%, 28%, 32%, 33%, 37%, 37.2%, 37.5%, 37.8%, 38%, 42%, 43%, or 48%;
(7) the transdermal penetration enhancer is one or more selected from an ester reagent, a surfactant, an ether reagent, a bile salt reagent, an alcohol reagent, a terpenoid reagent, a sulfoxide reagent, and a cyclodextrin reagent; the ether reagent is preferably one or more selected from diethylene glycol monoethyl ether, polyoxyethylene lauryl ether, and polyethylene glycol cetyl ether; the bile salt reagent is preferably selected from deoxycholate and/or glycocholate; the ester reagent is preferably one or more selected from propylene glycol monolaurate, glyceryl mono and dicaprylocaprate, monocaprylin, ethyl oleate, isopropyl myristate, glyceryl monocaprylocaprate, propylene glycol monocaprylate, polyglycerol oleate, D-α-tocopheryl polyethylene glycol succinate, and soybean phospholipid; the surfactant is preferably one or more selected from Tween 20, Tween 40, Tween 60, Tween 80, Span 20, Span 40, Span 60, Span 80, Span 85, sodium dodecyl sulfate, polyoxyethylene (35) castor oil, and polyoxyethylene 40 hydrogenated castor oil; the alcohol reagent is preferably an aliphatic alcohol reagent or polyol reagent; the aliphatic alcohol reagent is preferably one or more selected from ethanol, oleyl alcohol, and octyl dodecanol; the polyol reagent is preferably propylene glycol; the terpenoid reagent is preferably menthol; the sulfoxide reagent is preferably dimethyl sulfoxide; the cyclodextrin reagent is preferably hydroxypropyl-β-cyclodextrin;
(8) the transdermal penetration enhancer is present in the pharmaceutical composition B in a mass percentage of 40% to 80%, preferably 50% to 70%, for example, 49%, 54%, 59%, 64%, or 69%.

8. The pharmaceutical composition B according to claim 7, wherein the pharmaceutical composition B satisfies one or more of the following conditions:
(1) the vehicle is one or more selected from water, dimethyl sulfoxide, propylene glycol, ethanol, glycerol, and polyethylene glycol 400, preferably one or more selected from dimethyl sulfoxide, polyethylene glycol 400, propylene glycol, and ethanol, more preferably is a mixed vehicle of dimethyl sulfoxide, propylene glycol, and polyethylene glycol 400, or a mixed vehicle of dimethyl sulfoxide and propylene glycol;
(2) when the vehicle is a mixed vehicle of an alcohol vehicle and a sulfoxide vehicle, and the alcohol vehicle is propylene glycol, then the mass ratio of propylene glycol to the sulfoxide vehicle is 3:1 to 9:1; preferably 4:1 to 8:1, for example, 4.6:1, 5.6:1, 6.6:1, or 7.6:1;
(3) when the vehicle is a mixed vehicle of a sulfoxide vehicle and an alcohol vehicle, and the alcohol vehicle is propylene glycol and polyethylene glycol 400, then the mass ratio of propylene glycol to the sulfoxide vehicle is 10:1 to 1:1, preferably 7:1 to 4:1, for example, 6.4:1 or 5.4:1; the mass ratio of polyethylene glycol 400 to the sulfoxide vehicle is 0.5:1 to 5:1, preferably from 1:1 to 3:1, for example, 1:1 or 2:1;
(4) the transdermal penetration enhancer is one or more selected from diethylene glycol monoethyl ether, hydroxypropyl-β-cyclodextrin, Tween 20, Tween 40, Tween 60, Tween 80, Span 20, Span 40, Span 60, Span 80, Span 85, deoxycholate, glycocholate, propylene glycol monolaurate, glyceryl mono and dicaprylocaprate, monocaprylin, ethyl oleate, isopropyl myristate, glyceryl monocaprylocaprate, propylene glycol monocaprylate, sodium dodecyl sulfate, polyoxyethylene lauryl ether, polyethylene glycol cetyl ether, menthol, polyglycerol oleate, polyoxyethylene (35) castor oil, oleyl alcohol, octyl dodecanol, polyoxyethylene 40 hydrogenated castor oil, D-α-tocopheryl polyethylene glycol succinate, and soybean phospholipid;
preferably, the transdermal penetration enhancer is one or more selected from diethylene glycol monoethyl ether, hydroxypropyl-β-cyclodextrin, Tween 80, propylene glycol monolaurate, glyceryl mono and dicaprylocaprate, monocaprylin, ethyl oleate, glyceryl monocaprylocaprate, and propylene glycol monocaprylate, for example, a mixed transdermal penetration enhancer of diethylene glycol monoethyl ether and hydroxypropyl-β-cyclodextrin; a mixed transdermal penetration enhancer of diethylene glycol monoethyl ether, Tween 80, and propylene glycol monolaurate; a mixed transdermal penetration enhancer of diethylene glycol monoethyl ether and glyceryl mono and dicaprylocaprate; a mixed transdermal penetration enhancer of diethylene glycol monoethyl ether and glyceryl monocaprylocaprate; a mixed transdermal penetration enhancer of diethylene glycol monoethyl ether, Tween 80, and monocaprylin; a mixed transdermal penetration enhancer of diethylene glycol monoethyl ether and monocaprylin; a mixed transdermal penetration enhancer of diethylene glycol monoethyl ether and ethyl oleate; a mixed transdermal penetration enhancer of diethylene glycol monoethyl ether and propylene glycol monolaurate; a transdermal penetration enhancer of diethylene glycol monoethyl ether, glyceryl mono and dicaprylocaprate, and glyceryl monocaprylocaprate; a mixed transdermal penetration enhancer of diethylene glycol monoethyl ether and propylene glycol monocaprylate; a mixed transdermal penetration enhancer of diethylene glycol monoethyl ether, monocaprylin, and glyceryl monocaprylocaprate; a mixed transdermal penetration enhancer of diethylene glycol monoethyl ether, glyceryl mono and dicaprylocaprate, and monocaprylin; or a mixed transdermal penetration enhancer of diethylene glycol monoethyl ether, monocaprylin, and propylene glycol monocaprylate;
the transdermal penetration enhancer is more preferably a mixed transdermal penetration enhancer of diethylene glycol monoethyl ether and propylene glycol monocaprylate, or a mixed transdermal penetration enhancer of diethylene glycol monoethyl ether, monocaprylin, and propylene glycol monocaprylate;
(5) when the transdermal penetration enhancer is a mixed transdermal penetration enhancer of diethylene glycol monoethyl ether and hydroxypropyl-β-cyclodextrin, then the mass ratio of diethylene glycol monoethyl ether to hydroxypropyl-β-cyclodextrin is 2:1 to 4:1, for example, 3.27:1;
(6) when the transdermal penetration enhancer is a mixed transdermal penetration enhancer of diethylene glycol monoethyl ether, Tween 80, and propylene glycol monolaurate, then the mass ratio of diethylene glycol monoethyl ether to propylene glycol monolaurate is 8:1 to 10:1, for example, 9.8:1; the mass ratio of Tween 80 to propylene glycol monolaurate is 2:1 to 4:1, for example, 3:1;
(7) when the transdermal penetration enhancer is a mixed transdermal penetration enhancer of diethylene glycol monoethyl ether and glyceryl mono and dicaprylocaprate, then the mass ratio of diethylene glycol monoethyl ether to glyceryl mono and dicaprylocaprate is 4:1 to 10:1, for example, 4.9:1 or 9.8:1;
(8) when the transdermal penetration enhancer is a mixed transdermal penetration enhancer of diethylene glycol monoethyl ether and glyceryl monocaprylocaprate, then the mass ratio of diethylene glycol monoethyl ether to glyceryl monocaprylocaprate is 8:1 to 10:1, for example, 9.8:1;
(9) when the transdermal penetration enhancer is a mixed transdermal penetration enhancer of diethylene glycol monoethyl ether, Tween 80, and monocaprylin, then the mass ratio of diethylene glycol monoethyl ether to monocaprylin is 8:1 to 10:1, for example, 9.8:1; the mass ratio of Tween 80 to monocaprylin is 0.5:1 to 2:1, for example, 1:1;
(10) when the transdermal penetration enhancer is a mixed transdermal penetration enhancer of diethylene glycol monoethyl ether and monocaprylin, then the mass ratio of diethylene glycol monoethyl ether to monocaprylin is 4:1 to 10:1, for example, 4.9:1 or 9.8:1;
(11) when the transdermal penetration enhancer is a mixed transdermal penetration enhancer of diethylene glycol monoethyl ether and ethyl oleate, then the mass ratio of diethylene glycol monoethyl ether to ethyl oleate is 8:1 to 10:1, for example, 9.8:1;
(12) when the transdermal penetration enhancer is a mixed transdermal penetration enhancer of diethylene glycol monoethyl ether and propylene glycol monolaurate, then the mass ratio of diethylene glycol monoethyl ether to propylene glycol monolaurate is 8:1 to 10:1, for example, 9.8:1;
(13) when the transdermal penetration enhancer is a mixed transdermal penetration enhancer of diethylene glycol monoethyl ether, glyceryl mono and dicaprylocaprate, and glyceryl monocaprylocaprate, the mass ratio of diethylene glycol monoethyl ether to glyceryl monocaprylocaprate is 8:1 to 10:1, for example, 9.8:1; the mass ratio of glyceryl mono and dicaprylocaprate to glyceryl monocaprylocaprate is 0.5:1 to 2:1, for example, 1:1;
(14) when the transdermal penetration enhancer is a mixed transdermal penetration enhancer of diethylene glycol monoethyl ether and propylene glycol monocaprylate, then the mass ratio of diethylene glycol monoethyl ether to propylene glycol monocaprylate is 3:1 to 6:1, for example, 4.9:1;
(15) when the transdermal penetration enhancer is a mixed transdermal penetration enhancer of diethylene glycol monoethyl ether, monocaprylin, and glyceryl monocaprylocaprate, the mass ratio of diethylene glycol monoethyl ether to glyceryl monocaprylocaprate is 8:1 to 10:1, for example, 9.8:1; the mass ratio of monocaprylin to glyceryl monocaprylocaprate is 0.5:1 to 2:1, for example, 1:1;
(16) when the transdermal penetration enhancer is a mixed transdermal penetration enhancer of diethylene glycol monoethyl ether, glyceryl mono and dicaprylocaprate, and monocaprylin, the mass ratio of diethylene glycol monoethyl ether to monocaprylin is 8:1 to 10:1, for example, 9.8:1; the mass ratio of glyceryl mono and dicaprylocaprate to monocaprylin is 0.5:1 to 2:1, for example, 1:1;
(17) when the transdermal penetration enhancer is a mixed transdermal penetration enhancer of diethylene glycol monoethyl ether, monocaprylin, and propylene glycol monocaprylate, then the mass ratio of diethylene glycol monoethyl ether to propylene glycol monocaprylate is 8:1 to 10:1, for example, 9.8:1; the mass ratio of monocaprylin to propylene glycol monocaprylate is 0.5:1 to 2:1, for example, 1:1.

9. The pharmaceutical composition B according to any one of claims 5 to 8, wherein the pharmaceutical composition B satisfies one or more of the following conditions:
(1) the pharmaceutical composition B consists of the active ingredient A, the active ingredient B, the vehicle, and the transdermal penetration enhancer;
(2) the pharmaceutical composition B preferably consists of components shown in any of the following schemes:
scheme one: 1% of the compound of formula I, 2% of minoxidil, 49% of diethylene glycol monoethyl ether, 28% of propylene glycol, 5% of dimethyl sulfoxide, and 15% of hydroxypropyl-β-cyclodextrin;
scheme two: 1% of the compound of formula I, 2% of minoxidil, 49% of diethylene glycol monoethyl ether, 23% of propylene glycol, 5% of dimethyl sulfoxide, 15% of Tween 80, and 5% of propylene glycol monolaurate;
scheme three: 1% of the compound of formula I, 2% of minoxidil, 49% of diethylene glycol monoethyl ether, 38% of propylene glycol, 5% of dimethyl sulfoxide, and 5% of glyceryl mono and dicaprylocaprate;
scheme four: 1% of the compound of formula I, 2% of minoxidil, 49% of diethylene glycol monoethyl ether, 38% of propylene glycol, 5% of dimethyl sulfoxide, and 5% of glyceryl monocaprylocaprate;
scheme five: 1% of the compound of formula I, 2% of minoxidil, 49% of diethylene glycol monoethyl ether, 33% of propylene glycol, 5% of dimethyl sulfoxide, 5% of Tween 80, and 5% of monocaprylin;
scheme six: 1% of the compound of formula I, 2% of minoxidil, 49% of diethylene glycol monoethyl ether, 38% of propylene glycol, 5% of dimethyl sulfoxide, and 5% of monocaprylin;
scheme seven: 1% of the compound of formula I, 2% of minoxidil, 49% of diethylene glycol monoethyl ether, 38% of propylene glycol, 5% of dimethyl sulfoxide, and 5% of ethyl oleate;
scheme eight: 1% of the compound of formula I, 2% of minoxidil, 49% of diethylene glycol monoethyl ether, 38% of propylene glycol, 5% of dimethyl sulfoxide, and 5% of propylene glycol monolaurate;
scheme nine: 1% of the compound of formula I, 2% of minoxidil, 49% of diethylene glycol monoethyl ether, 33% of propylene glycol, 5% of dimethyl sulfoxide, and 10% of glyceryl mono and dicaprylocaprate;
scheme ten: 1% of the compound of formula I, 2% of minoxidil, 49% of diethylene glycol monoethyl ether, 33% of propylene glycol, 5% of dimethyl sulfoxide, 5% of glyceryl mono and dicaprylocaprate, and 5% of glyceryl monocaprylocaprate;
scheme eleven: 1% of the compound of formula I, 2% of minoxidil, 49% of diethylene glycol monoethyl ether, 33% of propylene glycol, 5% of dimethyl sulfoxide, and 10% of propylene glycol monocaprylate;
scheme twelve: 1% of the compound of formula I, 2% of minoxidil, 49% of diethylene glycol monoethyl ether, 33% of propylene glycol, 5% of dimethyl sulfoxide, and 10% of monocaprylin;
scheme thirteen: 1% of the compound of formula I, 2% of minoxidil, 49% of diethylene glycol monoethyl ether, 33% of propylene glycol, 5% of dimethyl sulfoxide, 5% of monocaprylin, and 5% of glyceryl monocaprylocaprate;
scheme fourteen: 1% of the compound of formula I, 2% of minoxidil, 49% of diethylene glycol monoethyl ether, 33% of propylene glycol, 5% of dimethyl sulfoxide, 5% of glyceryl mono and dicaprylocaprate, and 5% of monocaprylin;
scheme fifteen: 1% of the compound of formula I, 2% of minoxidil, 49% of diethylene glycol monoethyl ether, 33% of propylene glycol, 5% of dimethyl sulfoxide, 5% of monocaprylin, and 5% of propylene glycol monocaprylate;
preferably, the pharmaceutical composition B follows scheme eleven and scheme fifteen;
(3) the pharmaceutical composition B consists of 1% of the compound of formula I, 2% of minoxidil, 49% of diethylene glycol monoethyl ether, 33% of propylene glycol, 5% of dimethyl sulfoxide, 0 to 5% of monocaprylin, and 5 to 10% of propylene glycol monocaprylate.

10. A combined drug kit, comprising
a first container, comprising the first pharmaceutical composition according to claim 3 or 4; and
a second container, comprising the second pharmaceutical composition according to claim 3 or 4.

11. A preparation method of the pharmaceutical composition B according to any one of claims 5 to 9, wherein the preparation method comprises the following step: mixing the active ingredient A, the active ingredient B, and a pharmaceutically acceptable carrier, wherein the pharmaceutically acceptable carrier comprises a vehicle and a transdermal penetration enhancer;
preferably, when the vehicle comprises a sulfoxide vehicle, the preparation method for the pharmaceutical composition B comprises the following steps:
(1) mixing the active ingredient A, the active ingredient B, and the sulfoxide vehicle to obtain a mixture 1;
(2) mixing the mixture 1 from step (1) with the other vehicle and the transdermal penetration enhancer to obtain the pharmaceutical composition B;
more preferably, the reaction conditions of the preparation method satisfy one or more of the following conditions:
(1) the active ingredient A is a compound of formula I, and/or the active ingredient B is minoxidil;
(2) in step (2), the mixing is performed by adding the other vehicle and the transdermal penetration enhancer to the mixture 1 from step (1);
(3) in step (2), the mixing is performed by stirring until uniform.

12. A use of a compound of formula I, a pharmaceutically acceptable salt thereof, a solvate thereof, or a solvate of the pharmaceutically acceptable salt thereof, the combined drug according to claim 1 or 2, the pharmaceutical composition A according to claim 3 or 4, the pharmaceutical composition B according to any one of claims 5 to 9, or the combined drug kit according to claim 10, in the manufacture of a medicament for preventing, alleviating, or treating alopecia areata, wherein the use is not a use of a pharmaceutical composition C in the manufacture of a medicament for preventing, alleviating, or treating alopecia areata, the pharmaceutical composition C comprising the following components by mass fraction: 0.1% to 3% of the compound of formula I or a pharmaceutically acceptable salt thereof, 35% to 93% of a vehicle, and 5% to 63% of a transdermal penetration enhancer; the vehicle is one or more selected from water, glycerol, polyethylene glycol 400, dimethyl sulfoxide, ethyl acetate, propylene glycol, and ethanol; the transdermal penetration enhancer is one or more selected from diethylene glycol monoethyl ether, azone, urea, oleic acid, diisopropyl adipate, menthol, N-methylpyrrolidone, imidurea, propyl gallate, isopropyl myristate, cyclodextrin, sodium dodecyl sulfate, polyoxyethylene lauryl ether, polyethylene glycol cetyl ether, Tween 20, Tween 40, Tween 60, Tween 80, Span 20, Span 40, Span 60, Span 80, Span 85, deoxycholate, glycocholate, monocaprylin, glyceryl mono and dicaprylocaprate, propylene glycol monolaurate, propylene glycol monocaprylate, and glyceryl behenate;

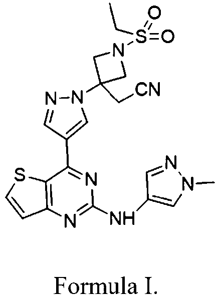

13. A method of treating alopecia areata, comprising administering to a subject in need thereof a therapeutically effective amount of a compound of formula I, a pharmaceutically acceptable salt thereof, a solvate thereof, or a solvate of the pharmaceutically acceptable salt thereof, the combined drug according to claim 1 or 2, the pharmaceutical composition A according to claim 3 or 4, the pharmaceutical composition B according to any one of claims 5 to 9, or the combined drug kit according to claim 10, wherein the method does not comprise administering to the subject in need thereof a therapeutically effective amount of a pharmaceutical composition C, the pharmaceutical composition C comprising the following components in mass fractions: 0.1% to 3% of the compound of formula I or a pharmaceutically acceptable salt thereof, 35% to 93% of a vehicle, and 5% to 63% of a transdermal penetration enhancer; the vehicle is one or more selected from water, glycerol, polyethylene glycol 400, dimethyl sulfoxide, ethyl acetate, propylene glycol, and ethanol; the transdermal penetration enhancer is one or more selected from diethylene glycol monoethyl ether, azone, urea, oleic acid, diisopropyl adipate, menthol, N-methylpyrrolidone, imidurea, propyl gallate, isopropyl myristate, cyclodextrin, sodium dodecyl sulfate, polyoxyethylene lauryl ether, polyethylene glycol cetyl ether, Tween 20, Tween 40, Tween 60, Tween 80, Span 20, Span 40, Span 60, Span 80, Span 85, deoxycholate, glycocholate, monocaprylin, glyceryl mono and dicaprylocaprate, propylene glycol monolaurate, propylene glycol monocaprylate, and glyceryl behenate;
